# EUROPEAN PATENT APPLICATION

(11) **EP 1 408 016 A1**
(43) Date of publication of application: **14.04.2004**
(21) Application number: 02022338.4
(22) Date of filing: 08.10.2002
(51) Int. Cl.: C03C 17/30, A61K 6/06, A61K 6/08

(54) **Glas powder for glas ionomer cement**

(71) Applicant: GC Corporation, Itabashi-ku, Tokyo 174 (JP)
(72) Inventor: Hirasawa, Michiko, Itabashi-ku, Tokyo (JP); Kato, Shinichi, Itabashi-ku, Tokyo (JP); Takuno, Makoto, Itabashi-ku, Tokyo (JP)
(74) Representative: Müller-Boré & Partner Patentanwälte

(57) **Abstract**

To provide a glass powder for glass ionomer cement, which has an effect for enhancing an adhesive force to a tooth structure as compared with the conventional glass powders for glass ionomer cement, and which is particularly suitable for a paste-like glass ionomer cement composition, the glass powder for glass ionomer cement is subjected to a surface treatment with at least one silane coupling agent having at least one group selected from a mercapto group, an amino group, and an epoxy group, or a surface treatment with at least one silane coupling agent having at least one group selected from a mercapto group, an amino group, and an epoxy group and with at least one vinyl group- or (meth)acryloyl group-containing silane coupling agent at the same time.

## Description

The present invention relates to a glass powder for glass ionomer cement to be used in the medicine, particularly the dentistry. More specifically, the present invention relates to a glass powder for glass ionomer cement, which has an effect for improving an adhesive force to a tooth structure as compared with the conventional glass powders for glass ionomer cement, and which is particularly suitable for a paste-like glass ionomer cement composition.

A glass ionomer cement is used by making an acid such as a polycarboxylic acid and a glass powder for glass ionomer cement reacted in the presence of water and curing the reacted mixture. This glass ionomer cement has characteristics such that it is extremely good in bioaffinity; its cured material is translucent and superior in aesthetics; it has a superior adhesive force to a tooth structure such as an enamel and a dentin; and that, when a fluoride is contained in the glass powder, it has a resisting action to caries by the fluoride. Accordingly, the glass ionomer cement is a dental material to be widely used in the dental field, including filling of a caries cavity, cementing of crowns, inlays, bridges, or orthodontic bands, lining of a cavity, sealing of a root canal, core construction, and preventive sealing.

Further, by adding a polymerizable resin component to the glass ionomer cement, not only the brittleness caused by moisture at the initial curing, that has hitherto been said to be a drawback of the glass ionomer cement, is inhibited, but also the mechanical strengths and physical properties such as adhesiveness to a tooth structure are improved, nowadays. In addition, resin-reinforced glass ionomer cements having superior adhesiveness to dental metals, resins, porcelains, etc. have also been developed. Moreover, glass ionomer cements, which can be quickly cured by visible light by using a photopolymerization catalyst as a catalyst for polymerizing a polymerizable resin component, have been developed, too. Thus, the utilization thereof has been a lot more expanded.

As described above, the glass ionomer cements have various characteristics and are placing on the market in various forms. However, general dental glass ionomer cements are constituted of a powder component and a liquid component, and therefore, one of disadvantages thereof is complication of operations such as measuring and mixing. For this reason, there has been developed a paste-like glass ionomer cement composition comprising two pastes of a paste comprised of a glass powder for glass ionomer cement and a polymerizable monomer (or water and a thickener) as major components and a paste comprised of a polycarboxylic acid and water (or a polymerizable monomer) as major components, with which a polymerization initiator is properly compounded according to the polymerization process of the polymerizable monomer.

However, though such a paste-like glass ionomer cement is superior in operation easiness during mixing, it was insufficient in adhesive force to a tooth structure as compared with the conventional glass ionomer cements using a mixture of a powder and a liquid.

The invention is aimed to overcome the above-described defects of the conventional paste-like glass ionomer cements and provide a glass powder for glass ionomer cement capable of obtaining a glass ionomer cement cured material having a high adhesive force to a tooth structure.

In order to achieve the above-described aim, the present inventors made extensive and intensive investigations. And, attention was paid to the matter that though a paste-like glass ionomer cement is superior in operation easiness, in order to maintain a strength necessary as the glass ionomer cement, it is required to contain a glass powder for glass ionomer cement in an amount as high as possible in the composition, and hence, wettability of the paste to a tooth surface is lowered so that the adhesive force to a tooth structure is insufficient. As a result, it has been found that, when the glass powder for glass ionomer cement is subjected to a surface treatment with a specific silane coupling agent, the wettability is enhanced so that the adhesive force of the glass ionomer cement to the tooth structure can be enhanced, leading to accomplishment of the present invention.

Specifically, the present invention is concerned with a glass powder for glass ionomer cement having been subjected to a surface treatment with at least one silane coupling agent having at least one group selected from a mercapto group, an amino group, and an epoxy group. It is preferred that 100 parts by weight of a surface-untreated glass powder for glass ionomer cement is subjected to a surface treatment with 0.1 to 10 parts by weight of at least one silane coupling agent having at least one group selected from a mercapto group, an amino group, and an epoxy group; and that 100 parts by weight of a surface-untreated glass powder for glass ionomer cement is subjected to a surface treatment with 0.1 to 10 parts by weight of at least one silane coupling agent having at least one group selected from a mercapto group, an amino group, and an epoxy group and a surface treatment with 0.1 to 10 parts by weight of at least one vinyl group- or (meth)acryloyl group-containing silane coupling agent at the same time.

Examples of a silane coupling agent having at least one group selected from a mercapto group, an amino group, and an epoxy group, which can be used for the glass powder for glass ionomer cement according to the present invention, include γ-mercaptopropyl trimethoxysilane as a silane coupling agent having at least one mercapto group; N-β(aminoethyl) γ-aminopropylmethyl dimethoxysilane, N-β(aminoethyl) γ-aminopropyl trimethoxysilane, N-β(aminoethyl) γ-aminopropyl triethoxysilane, γ-aminopropyl trimethoxysilane, γ-aminopropyl triethoxysilane, and N-phenyl-γ-aminopropyl trimethoxysilane as a silane coupling agent having at least one amino group; and γ-glycidoxypropyl trimethoxysilane, γ-glycidoxypropylmethyl diethoxysilane, γ-glycidoxypropyl triethoxysilane, and β-(3,4-epoxycyclohexyl)ethyl trimethoxysilane as a silane coupling agent having at least one epoxy group.

In the glass powder for glass ionomer cement according to the present invention, it is preferred that the surface treatment with at least one silane coupling agent having at least one group selected from a mercapto group, an amino group, and an epoxy group is carried out in a manner such that 100 parts by weight of a surface-untreated glass powder for glass ionomer cement'is subjected to a surface treatment with 0.1 to 10 parts by weight of at least one silane coupling agent having at least one group selected from a mercapto group, an amino group, and an epoxy group. When the amount of the at least one silane coupling agent having at least one group selected from a mercapto group, an amino group, and an epoxy group is less than 0.1 parts by weight, the desired effect is hardly obtained even when the surface treatment is carried out. On the other hand, when it exceeds 10 parts by weight, the function of the glass powder for glass ionomer cement to react with an acid tends to be inferior.

The glass powder for glass ionomer cement before the surface treatment in the glass powder for glass ionomer cement according to the present invention is a glass powder comprised of silica and alumina as major components. Specific examples include fluoroaluminosilicate glasses comprised of silica and alumina as major components and having, for example, calcium fluoride, aluminum fluoride, or aluminum phosphate mixed therewith, as disclosed in Japanese Patent Laid-Open No. 26925/1996. Of these fluoroaluminosilicate glasses is preferred a dental fluoroaluminosilicate glass powder containing 10 to 21 % by weight of Al³⁺, 9 to 21 % by weight of Si⁴⁺ and 1 to 20 % by weight of F- as components and further containing Sr²⁺ and Ca²⁺ in a total sum of 10 to 34 % by weight. Besides, any glass powders comprised of silica and alumina as major components and reactive with an acid can be used as the glass powder for glass ionomer cement before the surface treatment in the present invention. Also, an aluminosilicate glass powder comprised of silica and alumina as major components and having, for example, phosphorus pentoxide mixed therewith can be used.

With respect to the glass powder for glass ionomer cement according to the present invention, 100 parts by weight of a surface-untreated glass powder for glass ionomer cement may be subjected to a surface treatment with 0.1 to 10 parts by weight of at least one silane coupling agent having at least one group selected from a mercapto group, an amino group, and an epoxy group and a surface treatment with 0.1 to 10 parts by weight of at least one vinyl group- or (meth)acryloyl group-containing silane coupling agent at the same time. When the surface-untreated glass powder for glass ionomer cement is subjected to a surface treatment with these at least two silane coupling agents, the reduction of the physical properties of the cured material can be suppressed. As a result, the physical properties on an interface with an adherend are enhanced, leading to an increase in the adhesive force to the tooth structure. Examples of the polymerizable vinyl group- or (meth)acryloyl group-containing silane coupling agent that is used for the surface treatment include vinyl-based silane coupling agents such as vinyl trimethoxysilane, vinyl triethoxysilane, γ-methacryloxypropyl trimethoxysilane, γ-methacryloxypropylmethyl dimethoxysilane, vinyl trichlorosilane, and vinyl tris-(2-methoxyethoxy)silane; and unsaturated carboxylic acids such as methacrylic acid, acrylic acid, and maleic acid.

In the glass powder for glass ionomer cement according to the present invention, a process for treating the surface-untreated glass powder for glass ionomer cement with the silane coupling agent having at least one group selected from a mercapto group, an amino group, and an epoxy group can be carried out according to the conventional silane coupling treatment process. For example, is employable a process in which the silane coupling agent is dissolved in a solution such as ethanol, and the resulting solution is added to the glass powder for glass ionomer cement to form a dispersion, which is then heat treated at a temperature of about 90 to 160 °C for 0.5 to 5 hours, followed by allowing it to stand for cooling. In this process, a concentration of the silane coupling agent to be dissolved in ethanol is preferably 1 to 20 % by weight. When the concentration of the silane coupling agent to be dissolved in ethanol is less than 1 % by weight, the effect to be brought by the addition of the silane coupling agent is hardly obtained, whereas when it exceeds 20 % by weight, condensation among the silane coupling agent begins so that the dispersion tends to be hardly formed. In the case where the surface treatment is carried out using the silane coupling agent having at least one group selected from a mercapto group, an amino group, and an epoxy group, and the silane coupling agent having the vinyl group- or (meth)acryloyl group-containing silane coupling agent, there are employable a process in which the both silane coupling agents are mixed together with ethanol to carry out the silane treatment at the same time; and a process in which the silane coupling agents are separately dissolved in a solution such as ethanol, and the both solutions are added simultaneously to form a dispersion.

The surface-untreated glass powder for glass ionomer cement that is used for the glass powder for glass ionomer cement according to the present invention may be subjected to a surface treatment with an acid or a fluoride prior to the surface treatment with the silane coupling agent, in the same manner as in the conventional glass powder for glass ionomer cement. By the surface treatment with an acid or a fluoride, not only the fluidity of a cement slurry increases to improve the operation easiness, but also the curing can be made sharp. Examples of the acid to be used for this surface treatment include phosphoric acid, hydrochloric acid, pyrophosphoric acid, tartaric acid, citric acid, glutaric acid, malic acid, and acetic acid and also include acidic substance such as monobasic phosphates and dibasic phosphates. In addition, examples of the fluoride to be used for the surface treatment include aluminum fluoride, zinc fluoride, tin fluoride, zirconium fluoride, acidic sodium fluoride, and acidic potassium fluoride.

Since the glass powder for glass ionomer cement according to the present invention reacts with a polycarboxylic acid that has hitherto been used for the glass ionomer cement, in the presence of water and is cured, it can be used in the glass ionomer cement composition without particular limitations, like the conventional glass powder for glass ionomer cement. Accordingly, the glass powder for glass ionomer cement according to the present invention can be used together with known polycarboxylic acids or polyphosphonic acids that are used in the conventional dental glass ionomer cement compositions, such as polyacrylic acid, polymaleic acid, and vinylphosphonic acid, or copolymers thereof.

As a matter of course, the glass powder for glass ionomer cement according to the present invention is applicable to the conventional glass ionomer cement compositions, in which a powder and a liquid are mixed and cured, and resin-reinforced glass ionomer cements having a polymerizable monomer and a chemical polymerization catalyst or a photopolymerization catalyst compounded therewith. In these cases, there is an effect for enhancing an adhesive force of the glass ionomer cement cured material to the tooth structure, too. Incidentally, the glass powder for glass ionomer cement according to the present invention may be properly compounded with usually employed pigments, etc., if desired.

### (Adhesive strength test)

A tooth root portion of bovine mandibular anterior teeth was cut off, and after extirpation of pulp, an adherent enamel was exposed using an SiC paper of #600. To the enamel, was stuck a masking tape bored with a hole having a diameter of 3 mm. A silicone-made mold having an inner diameter of 5 mm and a height of 1 mm was placed on the bored position, and a paste-like glass ionomer cement prepared by mixing 1 g of a first paste and 1 g of a second paste, or a glass ionomer cement prepared by mixing 2 g of a powder and 1 g of a liquid, was filled therein. In the case where the glass ionomer cement to be filled is of a light-curing type, the surface of the test sample was brought into press contact with a celluloid plate and cured upon irradiation with light for 40 seconds by means of a visible light curing unit (a trade name: GC New Light VL-II, manufactured by GC Corporation), followed by placing in a thermostat chamber at a temperature of 37 °C and at a humidity of 100 %. In the case where the glass ionomer cement to be filled is not of a light-curing type, the surface of the test sample was brought into press contact with a celluloid plate and cured, followed by placing in a thermostat chamber at a temperature of 37 °C and at a humidity of 100 %. Twenty-four hours after placing in the thermostat chamber, the test sample was subjected to a tensile adhesive force test at a crosshead speed of 1 mm/min. using a universal tester (a trade name: Autograph, manufactured by Shimadzu Corporation). An average value of eight test samples and a standard deviation were obtained. The results obtained are summarized and shown in Table 1.

### (Preparation of surface-untreated glass powder for glass ionomer cement)

Twenty-two grams of aluminum oxide, 43 g of anhydrated silica, 12 g of calcium fluoride, 15 g of calcium phosphate, and 8 g of strontium carbonate were thoroughly mixed, and the mixture was molten while keeping in a high-temperature electric furnace at 1,200 °C for 5 hours. Thereafter, the melt was cooled and pulverized using a ball mill for 10 hours. The resulting product was made to pass through a 200-mesh (ASTM) sieve. The thus obtained powder was used as a surface-untreated glass powder for glass ionomer cement.

### (Silane treatment process of Examples 1 to 14)

### Example 1:

To 100 g of the surface-untreated glass powder for glass ionomer cement, was added 20 g of an ethyl alcohol solution of 5 % γ-aminopropyl trimethoxysilane. The mixture was thoroughly mixed in a mortar and subjected to a silane treatment by drying under the temperature condition as shown in Table 1 using a steam dryer.

### Examples 2 and 9:

To 100 g of the surface-untreated glass powder for glass ionomer cement was added 20 g of an ethyl alcohol solution of 10 % γ-aminopropyl trimethoxysilane. The mixture was thoroughly mixed in a mortar and subjected to a silane treatment by drying under the temperature condition as shown in Table 1 using a steam dryer.

### Examples 3 and 10:

To 100 g of the surface-untreated glass powder for glass ionomer cement was added 20 g of an ethyl alcohol solution of 20 % γ-aminopropyl trimethoxysilane. The mixture was thoroughly mixed in a mortar and subjected to a silane treatment by drying under the temperature condition as shown in Table 1 using a steam dryer.

### Examples 4 and 11:

To 100 g of the surface-untreated glass powder for glass ionomer cement was added 20 g of an ethyl alcohol solution of 10 % γ-aminopropyl trimethoxysilane and 10 % vinyl triethoxysilane. The mixture was thoroughly mixed in a mortar and subjected to a silane treatment by drying under the temperature condition as shown in Table 1 using a steam dryer.

### Examples 5 and 12:

To 100 g of the surface-untreated glass powder for glass ionomer cement was added 40 g of an ethyl alcohol solution of 20 % γ-glycidoxypropyl trimethoxysilane. The mixture was thoroughly mixed in a mortar and subjected to a silane treatment by drying under the temperature condition as shown in Table 1 using a steam dryer.

### Examples 6 and 13:

To 100 g of the surface-untreated glass powder for glass ionomer cement was added 20 g of an ethyl alcohol solution of 5 % γ-glycidoxypropyl trimethoxysilane and 5 % γ-methacryloxypropylmethyl dimethoxysilane. The mixture was thoroughly mixed in a mortar and subjected to a silane treatment by drying under the temperature condition as shown in Table 1 using a steam dryer.

### Examples 7 and 14:

To 100 g of the surface-untreated glass powder for glass ionomer cement was added 20 g of an ethyl alcohol solution of 10 % β-(3,4-epoxycyclohexyl)ethyl trimethoxysilane. The mixture was thoroughly mixed in a mortar and subjected to a silane treatment by drying under the temperature condition as shown in Table 1 using a steam dryer.

### Example 8:

To 100 g of the surface-untreated glass powder for glass ionomer cement was added 40 g of an ethyl alcohol solution of 20 % γ-mercaptopropyl trimethoxysilane. The mixture was thoroughly mixed in a mortar and subjected to a silane treatment by drying under the temperature condition as shown in Table 1 using a steam dryer.

### (Paste-based glass ionomer cement composition A (Examples 1 to 8))

Ten % by weight of hydroxyethyl methacrylate, 15 % by weight of glycidyl methacrylate, 15 % by weight of 2-hydroxy-1-acryloxy-3-methacryloxypropane di-2-methacryloxyethyl-2,2,4-triethylhexamethylene dicarbamate, and 60 % by weight of the glass powder for glass ionomer cement according to the present invention were mixed to prepare a first paste. On the other hand, 25 % by weight of an acrylic polymer having a weight-average molecular weight of 25,000, 30 % by weight of distilled water, 43 % by weight of a silane-treated silica sand powder prepared by adding 20 g of an ethyl alcohol solution of 10 % vinyl ethoxysilane to 100 g of finely powdered silica sand having a mean particle size of 2 µm, thoroughly stirring the mixture in a mortar, and then drying at 110 °C for 2 hours, and 2 % by weight of sodium benzenesulfonate, to prepare a second paste. From the first paste and the second paste, was prepared a paste-based glass ionomer cement composition A.

### (Paste-based glass ionomer cement composition B (Examples 9 to 11))

A paste-based glass ionomer cement composition B was prepared in the same manner as in the case of the paste-based glass ionomer cement composition A, except that the amount of the 2-hydroxy-1-acryloxy-3-methacryloxypropane di-2-methacryloxyethyl-2,2,4-triethylhexamethylene dicarbamate in the first paste was changed to 14 % by weight and that 1 % by weight of camphor quinone was added.

### (Glass ionomer cement composition (Examples 12 to 14))

A glass ionomer cement composition was prepared, which is cured upon mixing 3.0 g of the glass powder for glass ionomer cement according to the present invention with 1.0 g of a commercially available glass ionomer cement curing liquid (a trade name: Fuji I ILC Liquid, made by GC Corporation).

### Comparative Example 1:

A paste-based glass ionomer cement composition was prepared in the same manner as in the case of the paste-based glass ionomer cement composition A, except that a commercially available glass powder for glass ionomer cement (a trade name: Fuji IILC Powder, made by GC Corporation) was used as the glass powder for glass ionomer cement.

### Comparative Example 2:

A paste-based glass ionomer cement composition was prepared in the same manner as in the case of the paste-based glass ionomer cement composition A, except that a surface-untreated glass powder for glass ionomer cement was used as the glass powder for glass ionomer cement.

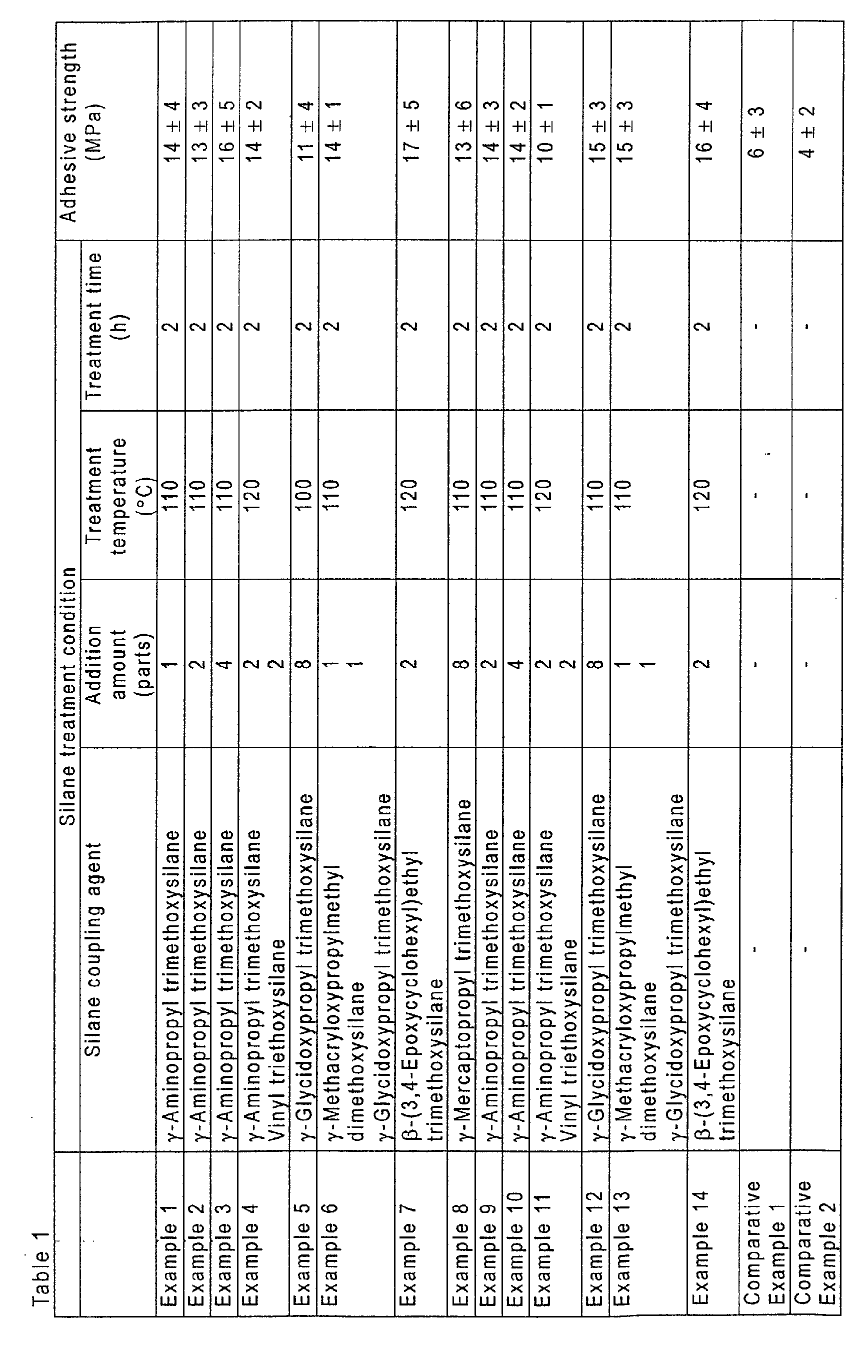

As described above in detail, the glass powder for glass ionomer cement according to the present invention has a superior adhesive force to a tooth structure as compared with the glass ionomer cement using a surface-untreated glass powder for glass ionomer cement, and hence, the present invention is greatly valuable in contributing to the dental remedy field.

While the present invention has been described in detail and with reference to specific embodiments thereof, it will be apparent to one skilled in the art that various changes and modifications can be made therein without departing from the spirit and scope thereof.

## Claims

1. A glass powder for glass ionomer cement having been subjected to a surface treatment with at least one silane coupling agent having at least one group selected from a mercapto group, an amino group, and an epoxy group.

2. The glass powder for glass ionomer cement according to claim 1, wherein 100 parts by weight of a surface-untreated glass powder for glass ionomer cement is subjected to a surface treatment with 0.1 to 10 parts by weight of at least one silane coupling agent having at least one group selected from a mercapto group, an amino group, and an epoxy group.

3. The glass powder for glass ionomer cement according to claim 1, wherein 100 parts by weight of a surface-untreated glass powder for glass ionomer cement is subjected to a surface treatment with 0.1 to 10 parts by weight of at least one silane coupling agent having at least one group selected from a mercapto group, an amino group, and an epoxy group and a surface treatment with 0.1 to 10 parts by weight of at least one vinyl group- or (meth)acryloyl group-containing silane coupling agent.
